# EUROPEAN PATENT APPLICATION

(11) **EP 4 512 953 A1**
(43) Date of publication of application: **26.02.2025**
(21) Application number: 24185604.6
(22) Date of filing: 01.07.2024
(51) Int. Cl.: D06N 3/00, C08L 5/04, C12N 1/14, D01F 4/00, D21H 13/34, D21H 23/20, D21H 13/30, A01G 18/00, D01F 1/10

(54) **ARTIFICIAL LEATHER AND MANUFACTURE METHOD THEREOF**

(30) Priority: 28.07.2023 TW 112128505
(71) Applicant: TAIWAN TEXTILE RESEARCH INSTITUTE, New Taipei City (TW)
(72) Inventor: CHANG, Haw-Jer, New Taipei City (TW); KUSUMA, Ricky Indra, New Taipei City (TW); HSU, Hui-Ju, New Taipei City (TW); CHOU, Hsin-Ying, New Taipei City (TW)
(74) Representative: Winter, Brandl - Partnerschaft mbB

(57) **Abstract**

An artificial leather is provided in some embodiments of the present disclosure, including: an artificial bio-nutritional fiber layer and a mycelium layer. The artificial bio-nutritional fiber layer includes a first surface, a second surface opposite to the first surface and a plurality of holes, in which the plurality of holes extends from the first surface to the second surface. The mycelium layer encapsulates the first surface and the second surface of the artificial bio-nutritional fiber layer, and extends from the first surface to the second surface through the plurality of holes. A manufacture method of an artificial leather is further provided in some embodiments of the present disclosure.

## Description

### BACKGROUND

### Field of Invention

The present disclosure relates to an artificial leather and manufacture method thereof.

### Description of Related Art

With the improvement of standard of living, people's requirements for leather are getting higher and higher. Among them, the application of artificial leather has gradually broken through the limitations of genuine leather and is widely used in various industrial fields. In the field of artificial leather, compared with artificial leather made from plants, artificial leather made from hyphae has the advantages of beautiful appearance and soft touch. Therefore, artificial leather made from hyphae is very popular in the market.

However, the existing artificial leather process made of hyphae includes the coating method, which uses chemical glue to bond hyphae and fiber layers. Therefore, there are limitations of insufficient structural strength and softness.

Therefore, how to provide artificial leather that improves structural strength and softness is a problem to be solved.

### SUMMARY

The present disclosure provides an artificial leather and a manufacture method of an artificial leather, an artificial bio-nutritional fiber layer including a nutritional material is served as a grown base of mycelium and allows mycelium clings along an artificial bio-nutritional fiber layer and through holes of the artificial bio-nutritional fiber layer, further encapsulating a plurality of surfaces, thereby providing an artificial leather with better structure strength (such as ultimate tensile strength) and softness.

Some embodiments of the present disclosure provide an artificial leather, including an artificial bio-nutritional fiber layer and a mycelium layer. The artificial bio-nutritional fiber layer includes a first surface, a second surface opposite to the first surface and a plurality of holes, in which the plurality of holes extend from the first surface to the second surface. The mycelium layer encapsulates the first surface and the second surface of the artificial bio-nutritional fiber layer and extends from the first surface to the second surface through the plurality of holes.

According to one embodiment of the present disclosure, a hole diameter of each of the plurality of holes is from 500 µm to 1000 µm.

According to one embodiment of the present disclosure, the artificial bio-nutritional fiber layer includes a skeleton material and a nutritional material, and a weight percentage of the skeleton material is from 10% to 90% and a weight percentage of the nutritional material is from 10% to 90% based on 100% by weight percentage of the artificial bio-nutritional fiber layer.

According to one embodiment of the present disclosure, the skeleton material includes alginate salt.

According to one embodiment of the present disclosure, the nutritional material includes vegetable protein, carbohydrate, inorganic salt, vitamin or a combination thereof.

According to one embodiment of the present disclosure, the vegetable protein includes soy protein, pea protein or a combination thereof.

Some embodiments of the present disclosure provide a manufacture method of an artificial leather, including: a mixing step, mixing a skeleton material and a first nutritional material to obtain a spinning solution; a wet spinning step, performing a wet spinning process on the spinning solution to obtain a spinning fiber; a paper-making step, performing a paper-making process on the spinning fiber to obtain an artificial bio-nutritional fiber layer; and a mycelium growth step, adding a second nutritional material and a mycelium solution including a mycelium on the artificial bio-nutritional fiber layer to make the mycelium grow on the artificial bio-nutritional fiber layer to obtain an artificial leather.

According to one embodiment of the present disclosure, a paper-making process includes: cutting the spinning fiber into a plurality of short fibers; distributing the plurality of short fibers in an aqueous solution to form a paper-making solution; and obtaining the artificial bio-nutritional fiber layer from the paper-making solution using a filter or a paper machine.

According to one embodiment of the present disclosure, the artificial bio-nutritional fiber layer includes a first surface, a second surface opposite to the first surface and a plurality of holes extending from the first surface to the second surface, wherein the mycelium completely encapsulates the artificial bio-nutritional fiber layer and extends from the first surface to the second surface through the plurality of holes.

According to one embodiment of the present disclosure, an ultimate tensile strength of the artificial leather is from 3 MPa to 105 MPa.

It is to be understood that both the foregoing general description and the following detailed description are examples and are intended to provide further explanation of the present disclosure as claimed.

### BRIEF DESCRIPTION OF THE DRAWINGS

Fig. 1 illustrates a flow chart of a manufacture method of an artificial leather.
Fig. 2A represents a comparison graph of hyphae length of groups N, O, P, and R on each culture day.
Fig. 2B represents photos of the culture mediums of groups N, O, P, and R when cultured for 10 days.
Fig. 3A represents a comparison graph of hyphae length of groups A, B, C, D, E, and F on each culture day.
Fig. 3B represents photos of the culture mediums of groups A, B, C, D, E and F when cultured for 10 days.
Fig. 4 represents a comparison graph of hyphae length of groups F, L and O on each culture day.
Fig. 5A and Fig. 5B respectively represents photos and electron microscope images of the gauze fibers magnified 200 times.
Fig. 5C and Fig. 5D respectively represents photos and electron microscope images of the artificial bio-nutritional fiber layer magnified 1000 times.
Figs 6A, 6B, and 6C are respectively electron microscope images of an upper surface, a cross section, and a lower surface of the artificial bio-nutritional fiber layer when the mycelium was cultured for 5 days.

### DETAILED DESCRIPTION

The present disclosure provides an artificial leather and a manufacture method of an artificial leather. An artificial bio-nutritional fiber layer with appropriate and uniform hole diameters and including nutritional material is manufactured by using a wet spinning step and a paper-making step, which allows mycelium to absorb nutrition of nutritional material, grow along biofibers of the artificial bio-nutritional fiber layer and through holes of the artificial bio-nutritional fiber layer and encapsulate the first surface and the second surface, thereby providing the artificial leather better structure strength and softness.

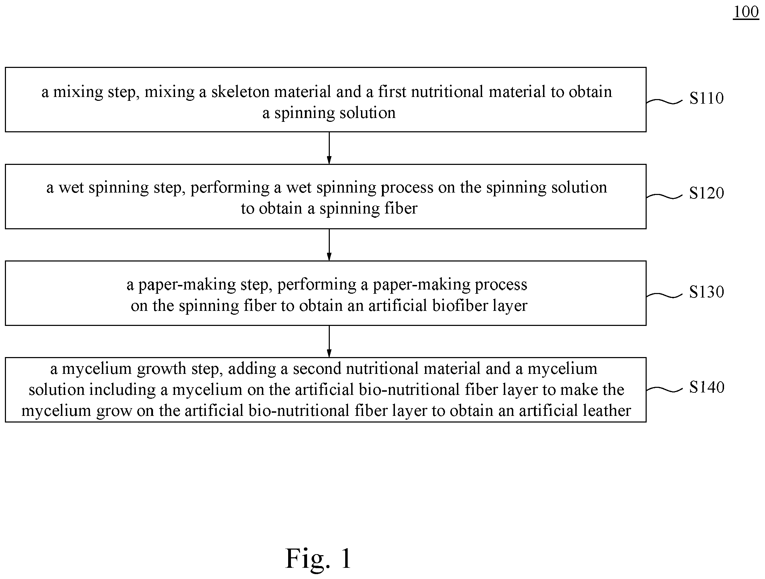

Please refer to Fig. 1, illustrating a flow chart of a manufacture method 100 of an artificial leather, including step 110 to step 140.

Step 110 is a mixing step, mixing a skeleton material and a first nutritional material to obtain a spinning solution.

In some embodiments, a skeleton material includes alginate salt (such as sodium alginate) for serving as a fiber skeleton of the artificial bio-nutritional fiber layer. In some embodiments, a first nutritional material includes vegetable protein (nitrogen source), carbohydrate (carbon source), inorganic salt, vitamin or a combination thereof, for providing mycelium with nutrition needed for grown, and a suitable first nutritional material can be selected according to the mycelium type selected in the subsequent procedure. It should be noted that compared with using single nutrient source, a first nutritional material can better promote the growth of mycelium when it includes a plurality of nutrient sources at the same time. For example, compared with adding only vegetable protein, adding vegetable protein and carbohydrate at the same time achieve the faster growth speed of hyphae.

In some embodiments, vegetable protein includes soy protein, pea protein or a combination thereof, providing a mycelium with nitrogen source needed for growth. In some embodiments, a molecular weight of vegetable protein is from 0.5×10⁹ dalton (Da) to 2.0×10⁹ Da, such as 0.5×10⁹ Da, 1.0×10⁹ Da, 1.5×10⁹ Da, 2.0×10⁹ Da or a value in the aforementioned intervals. If the molecular weight is too high, vegetable protein is not easy to be adsorbed by mycelium. If the molecular weight is too small, nutritional material is consumed too quickly. It can be understood that, compared with soy protein (molecular weight is 1.6×10⁹ Da), vegetable protein with small molecular weight (such as pea protein with the molecular weight of 1.0×10⁹ Da) increase nutrition absorption rate of the mycelium and the growth speed of hyphae. In some embodiments, carbohydrate includes monosaccharide (such as glucose, fructose or galactose, etc.), disaccharide (such as maltose, sucrose or lactose, etc) or polysaccharide (such as starch), in which when using monosaccharide with a smaller molecular weight, mycelium absorbs monosaccharide faster and the growth of mycelium can be better promoted. In some embodiments, inorganic salt includes phosphorus, potassium, calcium, sulfur or a combination thereof, providing nutritional substance needed for growth of mycelium. In some embodiments, vitamin includes vitamin A, vitamin B complex, vitamin C, or a combination thereof, in which vitamin B complex has better efficiency for accelerating mycelium growth.

In some embodiments, the step of mixing the skeleton material and the first nutritional material includes mixing the skeleton material, the first nutritional material and water to allow the skeleton material and the first nutritional material to be uniformly dissolved in the spinning solution.

In some embodiments, a weight ratio of the skeleton material and the first nutritional material is from 10:90 to 90:10, such as 10:90, 20:80, 30:70, 40:80, 50:70, 60:40, 70:30, 80:20, 90:10 or a value in the aforementioned intervals. If the weight ratio is too high, there will be insufficient nutrient sources, which will influence mycelium growth. If the weight ratio is too low, the fiber strength will be insufficient.

Step S120 is a wet spinning step, performing a wet spinning process on the spinning solution to obtain a spinning fiber. A spinning fiber manufactured by the wet spinning step includes the first nutritional material for providing nutrient source needed for the following growth of mycelium.

In some embodiments, a wet spinning process includes introducing the spinning solution into a coagulation bath to make the skeleton material and the first nutritional material precipitate and solidify to form a spinning fiber. In some embodiments, the coagulation bath includes a coagulation bath material and an organic solvent, in which the coagulation bath material includes chloride salts or sulfate salts, etc. The organic solvent includes ethanol or acetone. In one embodiment, a suitable coagulation bath material can be selected accordingly based on the selection of the skeleton material. For example, when the skeleton material is alginate salt and calcium chloride is selected as the coagulation bath material, the spinning fiber with better quality can be achieved.

Step S130 is a paper-making step, performing a paper-making process on the spinning fiber to obtain an artificial bio-nutritional fiber layer. Through a paper-making process, the artificial bio-nutritional fiber layer with the uniform hole sizes and an appropriate range of hole diameters can be obtained to allow the following mycelium to grow through the holes, reinforce the structure of the holes and cling along biofibers.

In some embodiments, the artificial bio-nutritional fiber layer is formed by crosslinking a plurality of biofibers, including a first surface, a second surface opposite to the first surface and a plurality of holes extending from the first surface to the second surface.

In some embodiments, a paper-making process includes: cutting the spinning fiber into a plurality of short fibers; distributing the plurality of short fibers in an aqueous solution to form a paper-making solution; and obtaining the artificial bio-nutritional fiber layer from the paper-making solution using a filter or a paper machine, in which using the filter can obtain the artificial bio-nutritional fiber layer one-time, and using the paper machine can obtain the artificial bio-nutritional fiber layer continuously. In some embodiments, a length of each of the plurality of short fibers is from 0.6 cm to 1.2 cm, such as 0.6 cm, 0.8 cm, 1 cm, 1.2 cm or a value in the aforementioned intervals. If the length is too short, the time required for the cutting procedure is too long; if the length is too long, the uniformity of the artificial bio-nutritional fiber layer is poor.

In some embodiments, the artificial bio-nutritional fiber layer includes a skeleton material and a first nutritional material, a weight percentage of the skeleton material is from 10% to 90% (such as 10%, 20%, 30%, 40%, 50%, 60%, 70%, 80%, 90% or a value in the aforementioned intervals) and a weight percentage of the nutritional material is from 10% to 90% (such as 10%, 20%, 30%, 40%, 50%, 60%, 70%, 80%, 90% or a value in the aforementioned intervals) based on 100% by weight percentage of the artificial bio-nutritional fiber layer. If the weight percentage of the skeleton material is too high or the weight percentage of the first nutritional material is too low, the nutrient source provided to the mycelium is insufficient and hyphae growth is influenced. If the weight percentage of the skeleton material is too low or the weight percentage of the first nutritional material is too high, the strength of the artificial bio-nutritional fiber layer is insufficient.

it is worth emphasized that through the paper-making process, the hole diameter can be controlled in an appropriate scope, such as the hole diameter of the artificial bio-nutritional fiber layer is controlled in a range of from 500 µm to 1000 µm, such as 500 µm, 600 µm, 700 µm, 800 µm, 900 µm, 1000 µm or a value in the aforementioned intervals. If the hole diameter is too big, the reinforcement effect of structural strength provided by the following growth of the hyphae along the holes is limited. If the hole diameter is too small, the hyphae cannot pass through the holes, but only grows on the specific surface of the artificial bio-nutritional fiber layer, and cannot extend to encapsulate each surface of the artificial bio-nutritional fiber layer, which limits the improvement of the enforcement of structure strength and softness.

Step 140 is a mycelium growth step, adding a second nutritional material and a mycelium solution including a mycelium on the artificial bio-nutritional fiber layer to make the mycelium grow on the artificial bio-nutritional fiber layer to obtain an artificial leather.

In some embodiments, the second nutritional material can be the same or similar to the first nutritional material and is used for supplying nutrition needed for mycelium growth, such as vegetable protein, carbohydrate, inorganic salt, vitamin or a combination thereof. In some embodiments, the mycelium includes Ganoderma genus mycelium, Pleurotus genus, Trametes genus mycelium, Brachyspora genus mycelium, Xylemporus genus mycelium, Polypore genus mycelium, Agaric genus mycelium, Tremella genus mycelium or a combination thereof.

In some embodiments, the mycelium grows along the biofiber of the artificial bio-nutritional fiber layer, clings on the biofiber and through holes between the biofibers, extends and encapsulates at least two surfaces, and forms the mycelium layer. That is, the mycelium layer of the artificial leather at least encapsulates the first surface and the second surface of the artificial bio-nutritional fiber layer and extends from the first surface to the second surface through the holes. In some embodiments, the mycelium layer encapsulates a plurality of surfaces of the artificial bio-nutritional fiber layer, but exposes a portion of the artificial bio-nutritional fiber layer. In some embodiments, the mycelium layer completely encapsulates the artificial bio-nutritional fiber layer (i.e., the mycelium layer encapsulates all surfaces of the artificial bio-nutritional fiber layer) to allow the artificial bio-nutritional fiber layer not to be exposed and to be buried in the mycelium layer. It can be understood that, as the extent of the mycelium layer encapsulating the artificial bio-nutritional fiber layer increases, the improvement effect on structural strength and softness of the artificial fibers is better.

In addition, it is worth emphasizing that compared with the mycelium leather only grows on a specific surface and cannot penetrate deeply into the holes of the fiber layer (for example, which is caused by the reason that the hole diameter of the fiber layer is too small or has no nutritional content, or caused by the chemical glue that blocks the hyphae extending to the fiber layer (such as impregnation method). The artificial bio-nutritional fiber layer of the artificial leather of the present disclosure can not only supply the mycelium nutritional material, but also has suitable hole diameters, which can allow the hyphae to pass through the holes, extend along the holes, and encapsulate a plurality of surfaces of the artificial bio-nutritional fiber layer. It can not only reinforce the strength defects in the holes to improve the structural strength, but also improve the softness of the artificial leather via the soft properties of the mycelium. In some embodiments, an ultimate tensile strength of the artificial leather is from 3 MPa to 105 MPa, such as 3 MPa, 5 MPa, 10 MPa, 20 MPa, 25 MPa, 30 MPa, 35 MPa, 40 MPa, 45 MPa, 50 MPa, 55 MPa, 60 MPa, 65 MPa, 70 MPa, 75 MPa, 80 MPa, 85 MPa, 90 MPa, 95 MPa, 100 MPa, 105 MPa or a value in the aforementioned intervals.

In the following description, several embodiments of the present disclosure will be listed to perform various analysis for verifying the functions of the present disclosure.

### Example 1-Selection of Materials of Artificial Bio-nutritional Fiber Layer

In order to test the better ingredients of the artificial bio-nutritional fiber layer for supplying hyphae growth, first of all, the agar gum culture mediums (the groups were numbered A to R and included 17 groups in total) with different additives and weight percentages were prepared according to the additive formula and the agar gum of each group in Table 1, in which a weight percentage of the agar gum was about 1% of the total weight of the culture medium.

Next, 5 mL of the mycelium solution (the mycelium solution concentration was 34.5 µm/ L) was applied to the agar gum culture medium of each group, and was cultured at 37°C for several days. Furthermore, hyphae growth status and hyphae length of each group were compared, and the results were described below.

**Table 1- Additive Formula of Culture Medium of Each Group of Culture**

| Number | Weight Percentage (%) | | | | |
|---|---|---|---|---|---|
| | Nutritional Material (Nitrogen Source) | | Fiber Skeleton | Nutritional Material (Carbohydrate Source) | Total |
| | Soy Protein (Soy Powder) | Pea Protein (Pea Powder) | Sodium Alginate | Glucose | |
| A | 0 | 0 | 0.7 | 0 | 0.7 |
| B | 0.3 | 0 | 0.7 | 0 | 1.0 |
| C | 0.7 | 0 | 0.3 | 0 | 1.0 |
| D | 0.5 | 0 | 0.5 | 0 | 1.0 |
| E | 0.3 | 0 | 0.3 | 0.4 | 1.0 |
| F | 0.2 | 0 | 0.3 | 0.5 | 1.0 |
| G | 0.1 | 0 | 0.3 | 0.6 | 1.0 |
| H | 0 | 0.3 | 0.7 | 0 | 1.0 |
| I | 0 | 0.7 | 0.3 | 0 | 1.0 |
| J | 0 | 0.5 | 0.3 | 0.2 | 1.0 |
| K | 0 | 0.3 | 0.3 | 0.4 | 1.0 |
| L | 0 | 0.2 | 0.3 | 0.5 | 1.0 |
| M | 0 | 0.1 | 0.3 | 0.6 | 1.0 |
| N | 0 | 0 | 0.7 | 0.3 | 1.0 |
| O | 0 | 0 | 0.3 | 0.7 | 1.0 |
| P | 0 | 0 | 0 | 1 | 1.0 |
| R | 0 | 0 | 0.3 | 0 | 0.3 |

| | | | | | |
|---|---|---|---|---|---|
| Note 1: The molecular weight of soy protein was 1.6×10⁹ Da. Note 2: The molecular weight of pea protein was 1.0×10⁹ Da. | | | | | |

### 1. Whether Addition of Nutritional Material Affects Hyphae Growth

### 1.1-Influence of Fiber Skeleton and Nutritional Material (Carbohydrate Source) on Hyphae Growth

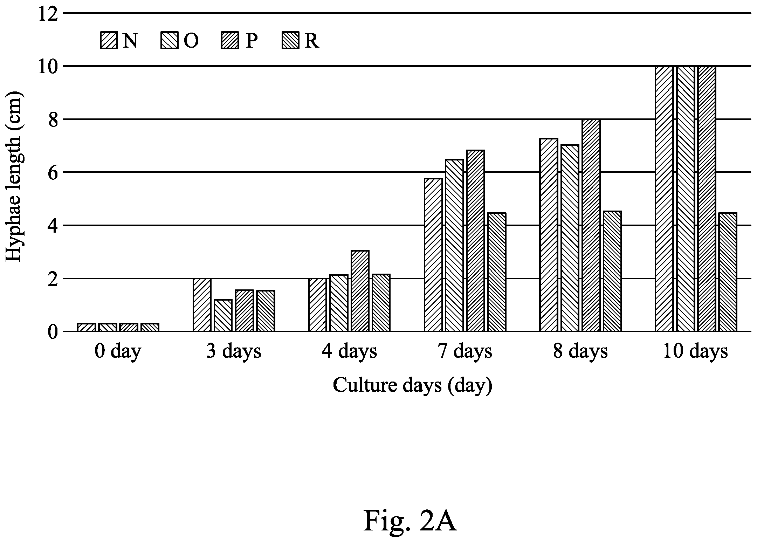

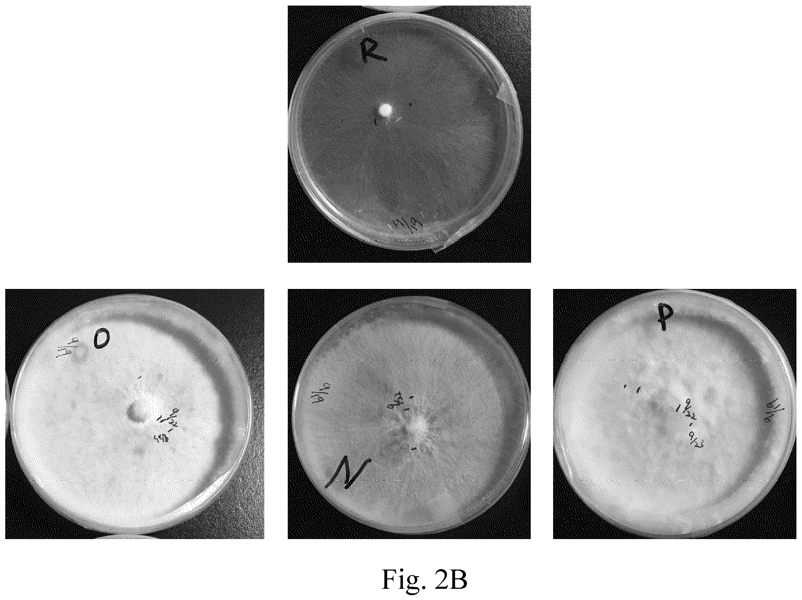

Please refer to Fig. 2A and Fig. 2B, which were respectively a comparison graph of hyphae length of groups N, O, P, and R (groups with different contents of sodium alginate and glucose and without the addition of soy protein and pea protein) on each culture day and photos of the culture mediums of groups N, O, P, and R when cultured for 10 days.

The results represented that (for example, please refer to the 7^{th} day to the 10^{th} day), the hyphae density and length of group R (sodium alginate of 0.3%) without adding any nutritional material were insufficient. Relatively, hyphae growth was clearly observed in group N, group O, and group P that added nutritional material (carbohydrate source, glucose). Among them, for example, please refer to the 4^{th} and 7^{th} day, as the addition amount of nutritional material was gradually increased from group N to group P (glucose in group N was 0.3%, glucose in group O was 0.7%, and glucose in group P was 1%), and the hyphae growth speed was also increased.

### 1.2- Influence of Nitrogen Source and Carbohydrate Source on Hyphae Growth

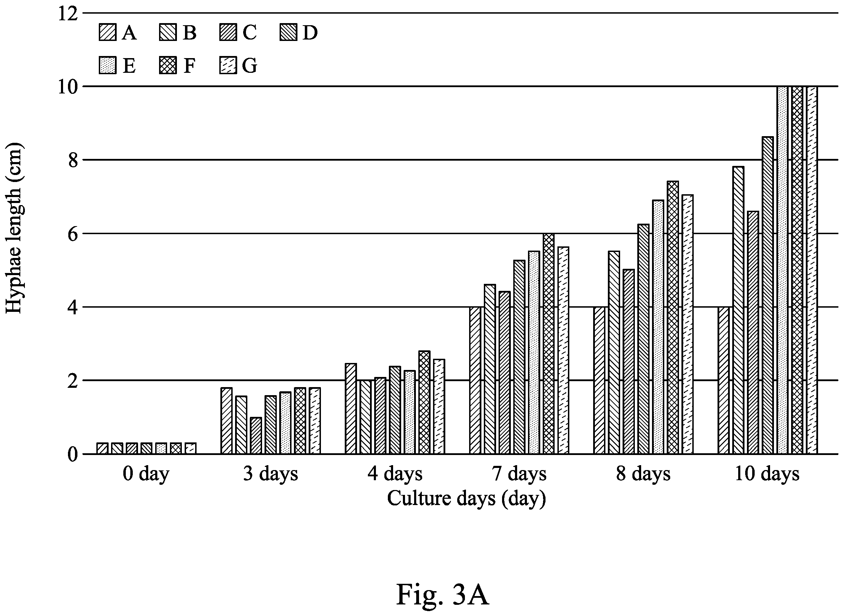

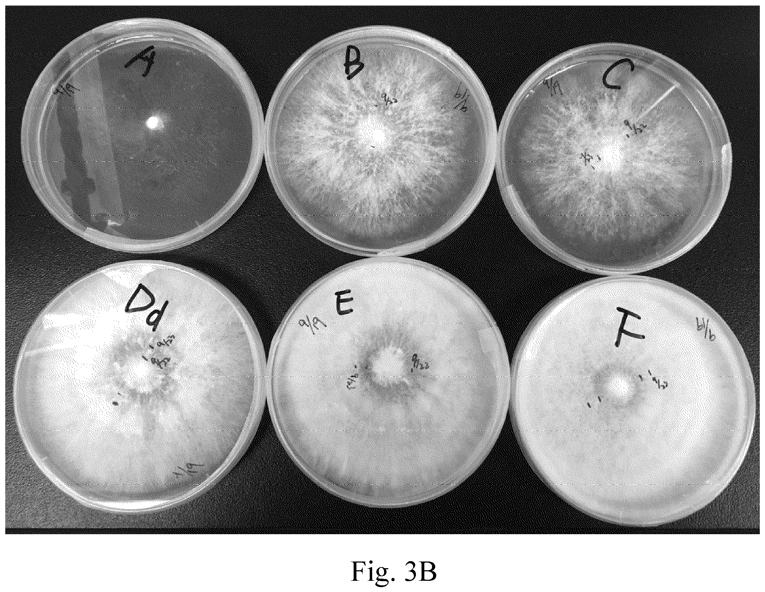

Please refer to Fig. 3A and Fig. 3B, which were respectively a comparison graph of hyphae length of groups A, B, C, D, E and F (groups with different contents of soy protein, sodium alginate and glucose and without the addition of pea protein) on each culture day and photos of the culture mediums of groups A, B, C, D, E and F when cultured for 10 days.

First of all, according to the comparison of soy protein, please refer to group A, B, C and D (soy protein of group A was 0%, soy protein of group B was 0.3%, soy protein of group C was 0.7%, soy protein of group D was 0.5%). On the 10^{th} day, though hyphae lengths of group B, group C and group D with addition of soy protein were longer than group A without addition of soy protein, hyphae length was not increased as the increase of soy protein. That is, compared with group A without the addition of nutrients, groups B, group C, and group D that added soy protein as nutrients all promoted hyphae growth. However, when soy protein was excessive (such as, the weight percentage of soy protein of group D was 0.5% and the weight ratio of soy protein and sodium alginate was 1:1), the increase of soy protein had limited effect on promotion of hyphae growth.

Furthermore, hyphae growth of adding carbohydrate source was further compared when the culture medium contained nitrogen source ( soy protein), please refer to group B, C, D, E, F and G (group B: 0.3% soy protein + 0% glucose, group C: 0.7% soy protein+0% glucose, group D: 0.5% soy protein+0% glucose, group E: 0.3% soy protein+ 0.4% glucose, group F: 0.2% soy protein+ 0.5% glucose, group G: 0.1% soy protein+ 0.6% glucose). On the 10^{th} day, it was observed that hyphae lengths of groups E, F and G that additionally added carbohydrate source were longer than groups B, C and D that only added nitrogen source.

For example, when comparing group B (without addition of glucose) and group E (addition of glucose) with the same content of soy protein, the growth speed of hyphae of group B was about 0.2 cm/ day to 0.4 cm/ day, the growth speed of hyphae of group E was about 0.6 cm/ day to 0.8 cm/ day, and the growth speed of hyphae differed by about 2.3 times. That is, group E with the addition of both of nitrogen source and carbohydrate source had quicker growth rate of hyphae.

Therefore, comparing with the situation that the nutritional material only includes nitrogen source, the situation that the nutritional material included both of nitrogen source and carbohydrate source supplemented the nutrition needed for hyphae growth and improved the growth behavior of hyphae.

### 1.3- Influence of Soy Protein, Pea Protein and Glucose on Hyphae Growth

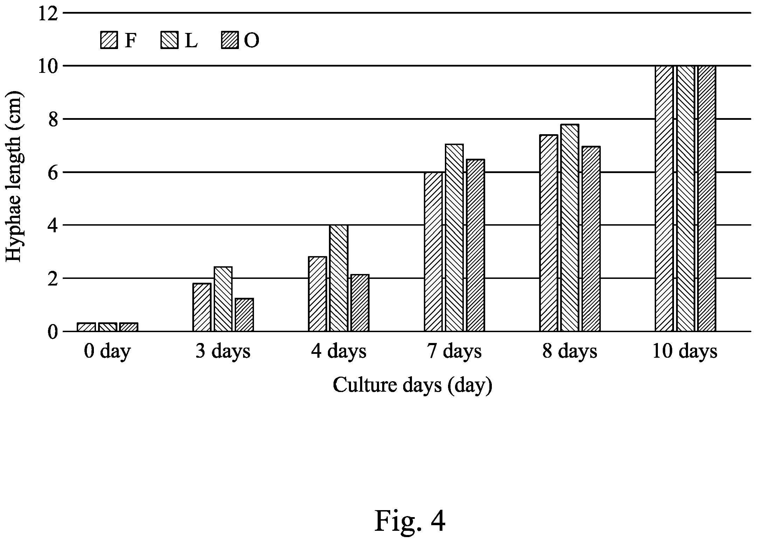

Please refer to Fig. 4, Fig. 4 was a comparison graph of hyphae length of groups F (0.2% soy protein+0.5% glucose ), L (0.2% pea protein+0.5% glucose ) and O (0.7% glucose ) on each culture day.

Please refer to the 3^{rd} day and the 4^{th} day of the rapid growth stage of hyphae, compared with group O (glucose) only with carbohydrate source, group F (soy protein+ glucose) and group L (pea protein+ glucose) had quicker growth speed. Therefore, the situation that nutritional material included both of nitrogen source and carbohydrate source had better promotion effect on hyphae growth.

Furthermore, please refer to the 3^{rd} day to the 8^{th} day. Compared with group F (soy protein+ glucose), group L (pea protein+ glucose) had a longer hyphae length and a faster growth rate. It can be understood that, compared with soy protein, pea protein had lower molecular weight and could be absorbed by the mycelium more easily. Therefore, comparing with the situation of soy protein+ glucose, the situation of pea protein+ glucose had better promotion effect on hyphae growth.

### Example 2-Manufacture of Artificial Leather and Properties Thereof

### 1. Manufacture of Artificial Bio-nutritional Fiber

To manufacture the artificial bio-nutritional fiber for fungus growth, soy protein and sodium alginate were selected to perform a wet spinning process together and obtain a spinning fiber. Then, the spinning fiber was used to perform a paper-making process, thereby obtaining an artificial bio-nutritional fiber. Detail steps were described as follows.

### 1.1-Manufacture of Spinning Fiber

First, sodium alginate (skeleton material), soy protein (nutritional material) and water were mixed, heated and stirred until dissolved to form a spinning solution, in which the weight percentage of the sum of sodium alginate and soy protein in the spinning solution was 10%. Furthermore, for testing manufacture conditions, the weight ratio of sodium alginate and soy protein was adjusted, the weight ratios of sodium alginate and soy protein in two groups of the spinning solutions were 50:50 or 35:65, respectively.

Furthermore, a wet spinning process was performed on the spinning solution, and then the spinning solution was introduced into a spinning machine to make the spinning solution flow out of the spinning nozzle and into a coagulation bath containing ethanol with 5% calcium chloride dissolved, and make sodium alginate and soy protein precipitate and solidify to form a spinning fiber.

### 1.2, Manufacture of Hand-made Paper

The spinning fibers were cut into short fibers with lengths of from 0.6 cm to 1.2 cm, the short fibers were distributed in an aqueous solution to form a paper-making solution. Furthermore, an artificial bio-nutritional fiber layer was obtained from the paper-making solution using a filter or a paper machine, and then dried and shaped.

### 2. Manufacture of Artificial Leather

The artificial bio-nutritional fiber layer obtained in point 1.2 was served as fungus growth carrier to manufacture an artificial leather. In particular, first of all, the artificial bio-nutritional fiber layer of 9 cm x9 cm was placed on the petri dish. Furthermore, 5 mL of the mycelium solution (the concentration of the mycelium solution: 34.5 mg/ L) containing the nutritional material (glucose was selected here) with the weight percentage of 10% was added to the surface of the artificial bio-nutritional fiber layer, then cultured for 14 days at 28°C to make the mycelium grow on the artificial bio-nutritional fiber layer to obtain an artificial leather, and then hyphae growth was observed with an electron microscope.

It should be noted that, theoretically, fungi with the mycelium was allowed to be used to prepare the artificial leather. Although Ganoderma lucidum was used in the current examples, it was not limited to this. In some other examples (for the sake of simplicity, not shown here), a similar growth trend was also represented when Pleurotus pleuricus, instead of Ganoderma lucidum, was used.

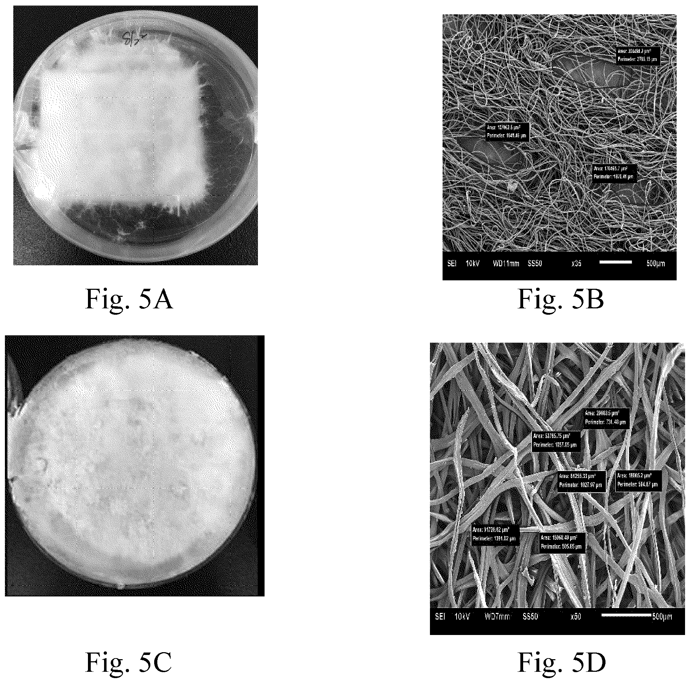

It could be understood that, an average hole diameter of gauze fibers manufactured by conventional techniques (please refer to Fig. 5A and Fig. 5B) was 2200 µm. Even if the mycelium grew on gauze fibers, the gauze fibers could not supply the nutrient source to the hyphae, therefore; the hyphae mainly grew on the upper surface of the gauze fibers and did not extend through the holes to cover other surfaces, not to mention encapsulating the entire gauze fibers. Furthermore, since the hole diameters of the gauze fibers were too big and the sizes were not uniform, the reinforcement effect of structural strength on gauze fibers by the hyphae was limited despite the hyphae grew on the gauze fibers.

Relatively, please refer to Fig. 5C and Fig. 5D. An average hole diameter of the artificial bio-nutritional fiber layer manufactured by the paper-making process was 700 µm, since the hole diameters were appropriate and the sizes were uniform, the hyphae could grow along the biofibers, through and filling the holes, and effectively reinforce structural strength. Therefore, when the hyphae grew on the artificial bio-nutritional fiber layer, the structure strength of the artificial leather was further enhanced and the softness was increased.

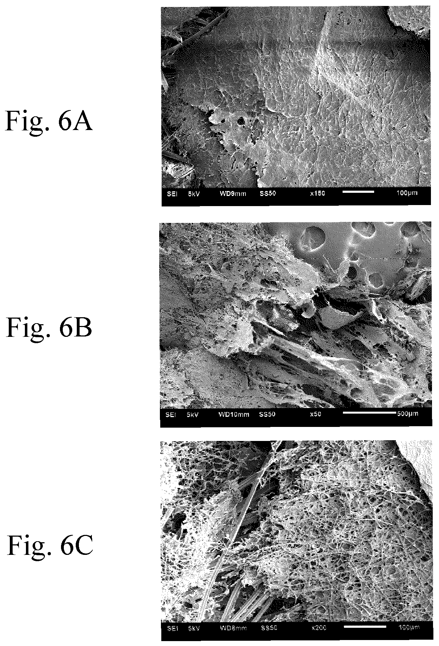

For example, please refer to Fig. 6A to Fig. 6C, respectively electron microscope images of an upper surface, a cross section, and a lower surface of the artificial bio-nutritional fiber layer when the mycelium was cultured for 5 days, in which the growth of the hyphae was observed on the upper surface, the cross section, and the lower surface, the hyphae passed through the holes in the artificial bio-nutritional fiber layer, extending from the upper surface, through and protruding from the lower surface.

Furthermore, although the figures were not shown here, as the culture days increased (for example, 15 days), it was observed that the hyphae grew along the biofibers and encapsulated all surfaces of the entire artificial bio-nutritional fiber layer, making only the mycelium layer be exposed to the outside among the artificial leather, and the artificial bio-nutritional fiber layer was buried in the mycelium layer.

### 3. Properties of Artificial Leather

To clarify the properties of the artificial leather with the mycelium layer, the artificial leathers manufactured by different conditions of the spinning solution mentioned before were continuously cultured for 9 days to 60 days (in the current artificial leather, the artificial bio-nutritional fiber layer was buried in the mycelium layer), and then various properties of the two groups of artificial leathers were measured, including biofiber diameter, fiber strength and tensile rate. The results were shown in Table 2.

**Table 2**

| Group | Formula of Spinning Solution | | Biofiber Diameter (µm) | Strength of Monofilament Fiber (cN/tex) | Tensile Rate (%) |
|---|---|---|---|---|---|
| | Soy Protein | Sodium Alginate | | | |
| 1 | 50% | 50% | from 40 to 60 | 3.4 | 3 |
| 2 | 65% | 35% | from 60 to 70 | 1.4 | 4.4 |

The result of Table 2 represented that when the content of soy protein in the spinning solution increased and the content of sodium alginate decreased, the fiber diameter increased and the strength of the monofilament fiber decreased, but the tensile rate increased.

Furthermore, the various properties of the artificial leather with different thicknesses in some examples of the present disclosure were illustrated in Table 3.

**Table 3**

| Properties | Value | |
|---|---|---|
| | Artificial Leather 1 | Artificial Leather 2 |
| Thickness | 0.5 mm | 0.9 mm |
| Basis Weight | 198 g/m² | 523 g/m² |
| Ultimate Tensile Strength | 3.4 MPa | 105 MPa |

## Claims

1. An artificial leather, **characterized by** comprising:
an artificial bio-nutritional fiber layer, comprising a first surface, a second surface opposite to the first surface and a plurality of holes extending from the first surface to the second surface; and
a mycelium layer, encapsulating the first surface and the second surface of the artificial bio-nutritional fiber layer and extending from the first surface to the second surface through the plurality of holes.

2. The artificial leather of claim 1, **characterized in that** a hole diameter of each of the plurality of holes is from 500 µm to 1000 µm.

3. The artificial leather of any one of claims 1 to 2, **characterized in that** the artificial bio-nutritional fiber layer comprises a skeleton material and a nutritional material, and a weight percentage of the skeleton material is from 10% to 90% and a weight percentage of the nutritional material is from 10% to 90% based on 100% by weight percentage of the artificial bio-nutritional fiber layer.

4. The artificial leather of claim 3, **characterized in that** the skeleton material comprises alginate salt.

5. The artificial leather of claim 3, **characterized in that** the nutritional material comprises vegetable protein, carbohydrate, inorganic salt, vitamin or a combination thereof.

6. The artificial leather of claim 5, **characterized in that** the vegetable protein comprises soy protein, pea protein or a combination thereof.

7. A manufacture method of an artificial leather, **characterized by** comprising:
a mixing step, mixing a skeleton material and a first nutritional material to obtain a spinning solution;
a wet spinning step, performing a wet spinning process on the spinning solution to obtain a spinning fiber;
a paper-making step, performing a paper-making process on the spinning fiber to obtain an artificial bio-nutritional fiber layer; and
a mycelium growth step, adding a second nutritional material and a mycelium solution comprising a mycelium on the artificial bio-nutritional fiber layer to make the mycelium grow on the artificial bio-nutritional fiber layer to obtain an artificial leather.

8. The manufacture method of the artificial leather of claim 7, **characterized in that** the paper-making process comprises:
cutting the spinning fiber into a plurality of short fibers;
distributing the plurality of short fibers in an aqueous solution to form a paper-making solution; and
obtaining the artificial bio-nutritional fiber layer from the paper-making solution using a filter or a paper machine.

9. The manufacture method of the artificial leather of any one of claims 7 to 8, **characterized in that** the artificial bio-nutritional fiber layer comprises a first surface, a second surface opposite to the first surface and a plurality of holes extending from the first surface to the second surface, wherein the mycelium completely encapsulates the artificial bio-nutritional fiber layer and extends from the first surface to the second surface through the plurality of holes.

10. The manufacture method of the artificial leather of any one of claims 7 to 9, **characterized in that** an ultimate tensile strength of the artificial leather is from 3 MPa to 105 MPa.
